# EUROPEAN PATENT APPLICATION

(11) **EP 4 393 422 A1**
(43) Date of publication of application: **03.07.2024**
(21) Application number: 24150055.2
(22) Date of filing: 02.01.2024
(51) Int. Cl.: A61B 17/56, A61B 17/15

(54) **METHOD FOR CORRECTING COMMON FOOT DISORDERS ASSOCIATED WITH DIABETES**

(30) Priority: 30.12.2022 US 202263436383 P
(71) Applicant: Dumfries Johnson, Daniel Mackey, Nassau, N.P. (BS)
(72) Inventor: Dumfries Johnson, Daniel Mackey, Nassau, N.P. (BS)
(74) Representative: Hoffmann Eitle

(57) **Abstract**

Embodiments relate to treating a foot disorder associated with diabetes by realigning and rebalancing abnormal destructive forces on a patient's feet. The method includes screening a patient with a foot deformity for diabetes, optionally correcting an ingrown toenail on said patient's foot and debriding ulcerated skin on said patient's foot, performing a tenotomy on each of said patient's toes with the deformity, performing a capsulotomy on each of said patient's toes with the deformity if said tenotomy did not correct said deformity, and performing an osteotomy on each metatarsus corresponding to each of said patient's toes with the deformity if said tenotomy and said capsulotomy did not correct said deformity.

## Description

### CROSS-REFERENCE TO RELATED APPLICATIONS

This application claims priority to U.S. Provisional Patent Application No. 63/436,383, which was filed on December 30, 2022. The entirety of this application is incorporated by reference herein.

### FIELD OF THE INVENTION

The present invention relates to treating feet of diabetic patients by realigning and rebalancing abnormal destructive forces on the feet.

### BACKGROUND OF THE INVENTION

Maladies of the human foot are some of the most common clinical sequalae of diabetes. Complications typically arise via the advent of diabetic neuropathy and diabetic angiopathy, leading to significantly reduced feeling and reduced circulation in the feet of diabetic patients. As such, many diabetic patients will begin walking with their weight improperly distributed on their feet. For example, abnormal weight bearing on the feet can lead to ulcers, deformities, hammertoes, hallux rigidus/valgus, and damaged or deformed metatarsals. In patients with abnormal weight bearing, the incidents of these complications are increased, and the frequency of morbidity and mortality is intense. Diabetic foot complications are often not effectively assessed and managed by doctors, either due to a lack of awareness or lack of new innovation in modern treatments. This lack of awareness is what delays necessary action. Doctors who do treat diabetic foot complications generally rely on pharmaceuticals as a primary focus of intervention. However, pharmaceutical intervention does not address the cause of the abnormal pressure exhibited in the foot, such as in the tendons, nerves, blood vessels, joints, and bones of the foot.

Failure to properly treat diabetic foot issues can lead to life changing amputations and premature death. The most common amputations are the lesser toes, the hallux, trans metatarsals, and below knee amputations. According to a recent NIH report, every 20 seconds someone in the world has an amputation. As mobility is a hallmark of being human, an amputation will change a life forever. Fifty percent of patients with below knee amputations tend to die within three to five years. This is a worse clinical outcome than most cancers.

It has been found that if the abnormal foot pressures, deformities, nerve sensation, and circulation can be improved, we see a clinically significant uptick in the positive outcome for these patients. If there is no ulceration, amputation is not required. In correcting the causative abnormalities, the recurrence rate of ulceration and amputation is likewise decreased. Accordingly, there is a need for a safe, simple, and effective method treating common foot disorders associated with diabetes.

### SUMMARY OF THE INVENTION

Embodiments relate to methods for correcting common foot disorders associated with diabetes. While previous treatments typically focus on treating symptoms, embodiments of the described method focus on the cause of the symptoms by releasing pressure on parts of a diabetic patient's foot that lead to resulting symptoms. The method includes performing sequential treatments until a condition is successfully treated. For example, the method may first treat one or more tendons, and if necessary, may then treat one or more capsules, and if necessary, may then treat one or more bones.

In an exemplary embodiment, a method for correcting a foot disorder comprises screening a patient with a foot deformity for diabetes; optionally correcting an ingrown toenail on said patient's foot; optionally debriding ulcerated skin on said patient's foot; performing a tenotomy on each of said patient's toes with the deformity; performing a capsulotomy on each of said patient's toes with the deformity if said tenotomy did not correct said deformity; and performing an osteotomy on each metatarsus corresponding to each of said patient's toes with the deformity if said tenotomy and said capsulotomy did not correct said deformity.

In some embodiments, the tenotomy comprises dividing a tendon flexor retinaculum.

In some embodiments, the tenotomy comprises dividing an extensor digitorum longus.

In some embodiments, the method further comprises manually stretching said patient's foot after the step of performing a tenotomy.

In some embodiments, the method further comprises performing a second tenotomy on each of said patient's toes with the deformity if said tenotomy did not correct said deformity.

In some embodiments, the capsulotomy comprises puncturing and dissecting a joint capsule.

In some embodiments, the method further comprises manually stretching said patient's foot after the step of performing a capsulotomy.

In some embodiments, the osteotomy comprises cutting a keyhole incision on the top of said patient's foot dorsal and proximal to a surgical neck of a metatarsal head of said metatarsus; and dissecting said metatarsus, wherein said dissection is performed at an angle such that said metatarsus is dissected perpendicularly to a centerline of said metatarsus.

In some embodiments, said angle ranges from 35 degrees to 45 degrees periosteum.

In some embodiments, said osteotomy is performed in a dorsal distal to plantar proximal plane to said metatarsal head.

In some embodiments, said osteotomy is a wedge osteotomy.

In some embodiments, said osteotomy further comprises the step of manually confirming by visual inspection or x-ray that each head of said metatarsus is lined up together.

In some embodiments, said deformity is hammertoe.

In some embodiments, the step of screening a patient with a foot deformity for diabetes comprises a screening method selected from the group consisting of glucose testing, hemoglobin A1C testing, testing to confirm a prior diagnosis, and combinations thereof.

In some embodiments, the step of screening the patient with the foot deformity for diabetes comprises a diagnostic testing method selected from the group consisting of neuropathic testing, angiopathic testing, visual analysis of the deformity, x-ray analysis of the deformity, and combinations thereof.

### BRIEF DESCRIPTION OF THE DRAWINGS

The above and other objects, aspects, features, advantages and possible applications of the present innovation will be more apparent from the following more particular description thereof, presented in conjunction with the following drawings. Like reference numbers used in the drawings may identify like components.
FIG. 1 depicts a tenotomy treating a toe of a diabetic patient in accordance with embodiments of the invention.
FIG. 2 depicts incisions of tenotomies treating toes of a diabetic patient in accordance with embodiments of the invention.
FIG. 3 depicts a tenotomy and/or capsulotomy on an ulcerated toe of a diabetic patient in accordance with embodiments of the invention.
FIG. 4 depicts a tenotomy and/or capsulotomy on an ulcerated toe of a diabetic patient in accordance with embodiments of the invention.
FIG. 5 depicts aligned toes of a diabetic patient after a tenotomies and capsulotomies in accordance with embodiments of the invention.
FIG. 6 depicts a tenotomy on an ulcerated toe of a diabetic patient in accordance with embodiments of the invention.
FIG. 7 depicts incisions of tenotomies on a foot of a diabetic patient in accordance with embodiments of the invention.
FIG. 8 depicts a tenotomy and/or capsulotomy on an ulcerated toe of a diabetic patient in accordance with embodiments of the invention.
FIG. 9 depicts a tenotomy and/or capsulotomy on an ulcerated toe of a diabetic patient in accordance with embodiments of the invention.
FIG. 10 depicts a surgeon stretching an ulcerated toe of a diabetic patient after a tenotomy and/or capsulotomy in accordance with embodiments of the invention.
FIG. 11 depicts an ulcer resulting from pressure of a hammer toe on a diabetic patient.
FIG. 12 depicts aligned toes of a diabetic patient after tenotomies and capsulotomies in accordance with embodiments of the invention.
FIG. 13 depicts debriding an ulcer on a toe of a diabetic patient in accordance with embodiments of the invention.
FIG. 14 depicts an ulcer resulting from pressure of a misaligned toe of a diabetic patient.
FIG. 15 depicts aligned toes of a diabetic patient after a tenotomy and/or capsulotomy in accordance with embodiments of the invention.
FIG. 16 depicts a misalignment of a toe and an ulcer caused by the misalignment of the toe of a diabetic patient prior to treatment.
FIG. 17 depicts proper alignment of a toe of a diabetic patient after treatment in accordance with embodiments of the invention.
FIG. 18 depicts proper alignment of a toe of a diabetic patient after treatment in accordance with embodiments of the invention.
FIG. 19 depicts debridement of an interdigital ulcer between the toes of a diabetic patient in accordance with embodiments of the invention.
FIG. 20 depicts misalignment of toes of a diabetic patient causing an ulcer between the toes.
FIG. 21 depicts misalignment of toes of a diabetic patient causing an ulcer between the toes.
FIG. 22 depicts misalignment of toes of a diabetic patient causing an ulcer between the toes.
FIG. 23 depicts a tenotomy and/or capsulotomy on an ulcerated toe of a diabetic patient in accordance with embodiments of the invention.
FIG. 24 depicts a tenotomy on a hammertoe in accordance with embodiments of the invention.
FIG. 25 depicts a tenotomy on a hammertoe in accordance with embodiments of the invention.
FIG. 26 depicts a corrected hammertoe in proper alignment after a tenotomy in accordance with embodiments of the invention.
FIG. 27 depicts forces created by tight tendons on a hammertoe.
FIG. 28 depicts forces created by tight tendons on a hammertoe.
FIG. 29 depicts forces created by tight tendons on a hammertoe.
FIG. 30 depicts directional forces of bones created by a hammertoe causing pressures of areas of a foot inside of a shoe.
FIG. 31 depicts an angle of an osteotomy on a metatarsus in accordance with embodiments of the invention.
FIG. 32 depicts an angle of an osteotomy on a metatarsus and a wedge osteotomy of a proximal phalange of a great toe in accordance with embodiments of the invention.
FIG. 33 illustrates a capsulotomy in accordance with embodiments of the invention.
FIG. 34 shows an exemplary method for correcting common foot disorders associated with diabetes.

The images in the drawings are simplified for illustrative purposes and are not depicted to scale. Within the descriptions of the figures, similar elements are provided similar names and reference numerals as those of the previous figure(s). The specific numerals assigned to the elements are provided solely to aid in the description and are not meant to imply any limitations (structural or functional) on the invention.

The appended drawings illustrate exemplary configurations of the invention and, as such, should not be considered as limiting the scope of the invention that may admit to other equally effective configurations. It is contemplated that features of one configuration may be beneficially incorporated in other configurations without further recitation.

### DETAILED DESCRIPTION OF THE INVENTION

The following description is of exemplary embodiments and methods of use that are presently contemplated for carrying out the present invention. This description is not to be taken in a limiting sense, but is made merely for the purpose of describing the general principles and features of various aspects of the present invention. The scope of the present invention is not limited by this description.

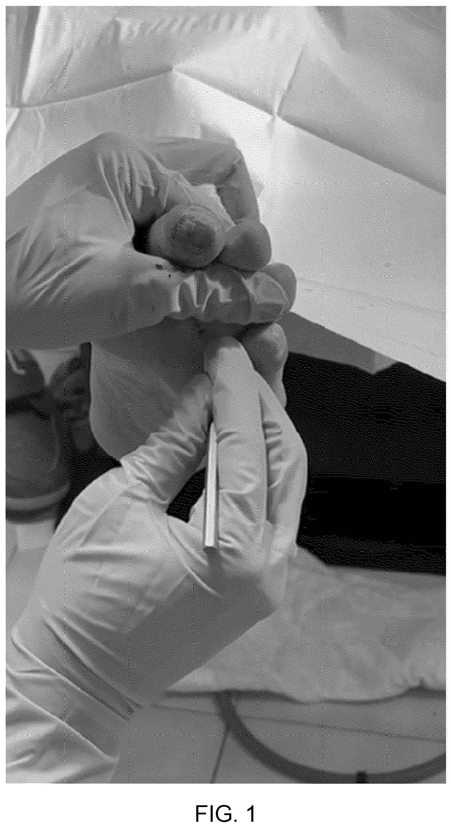

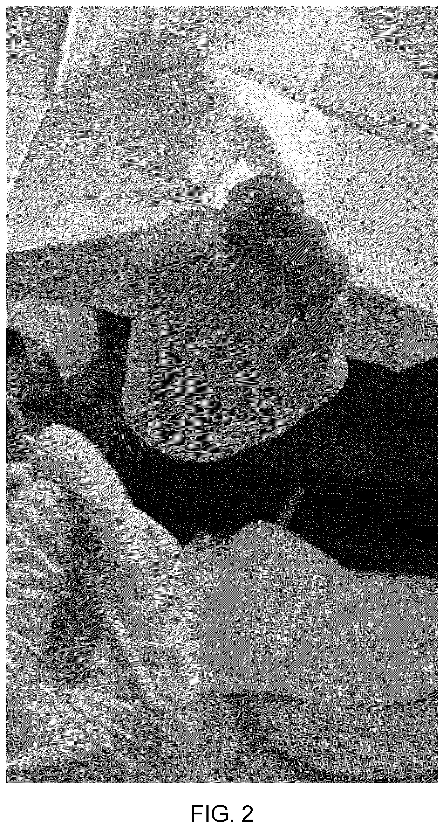

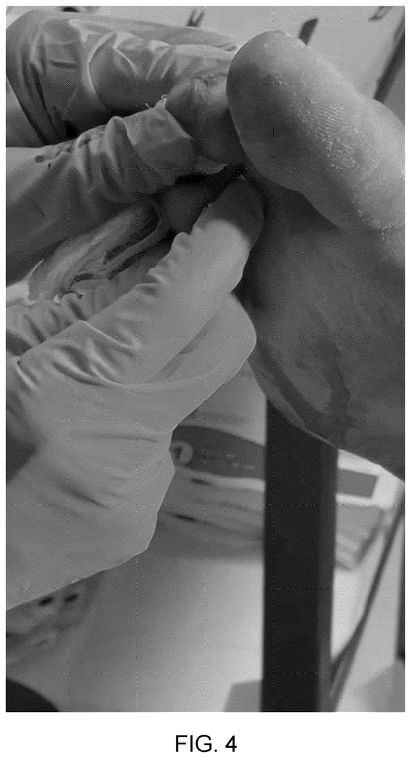

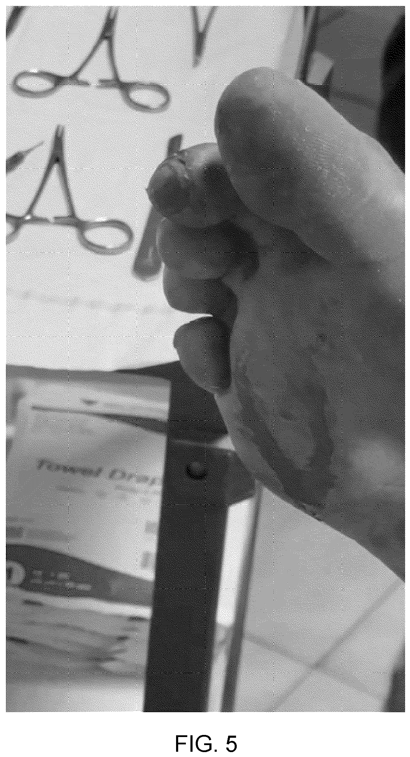

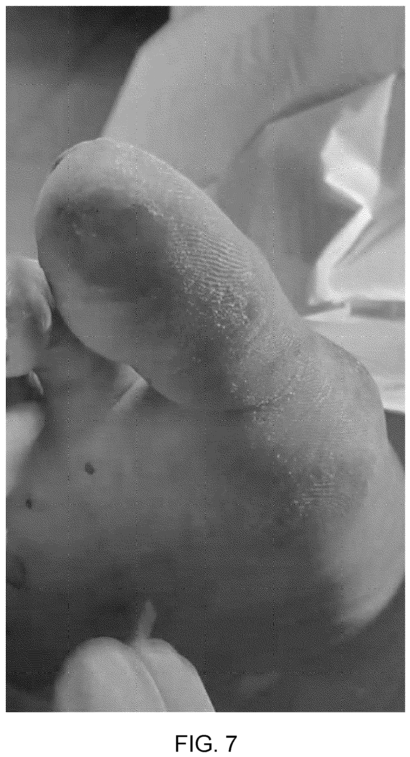

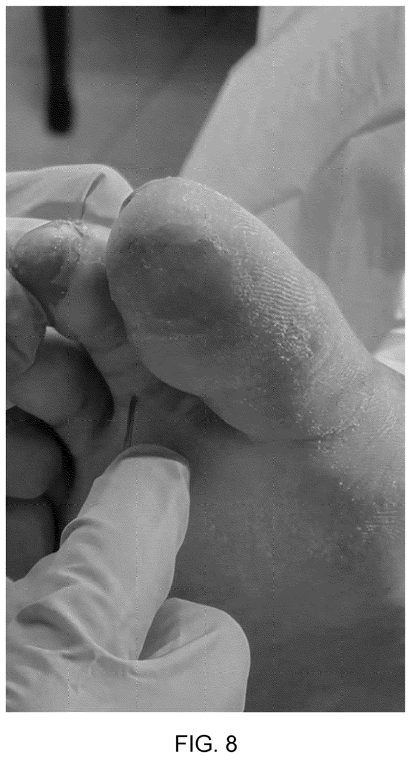

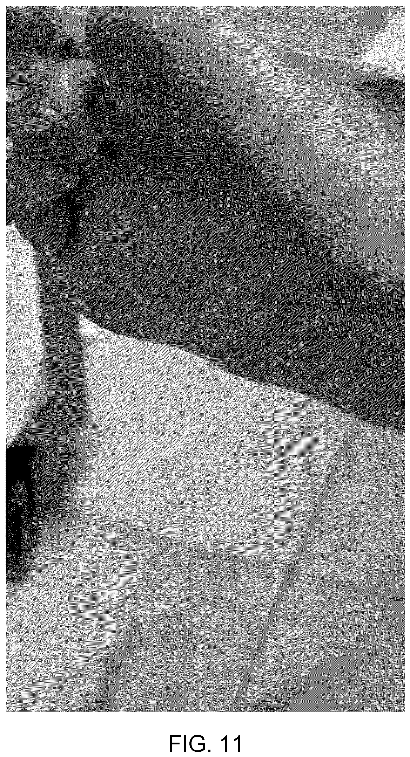

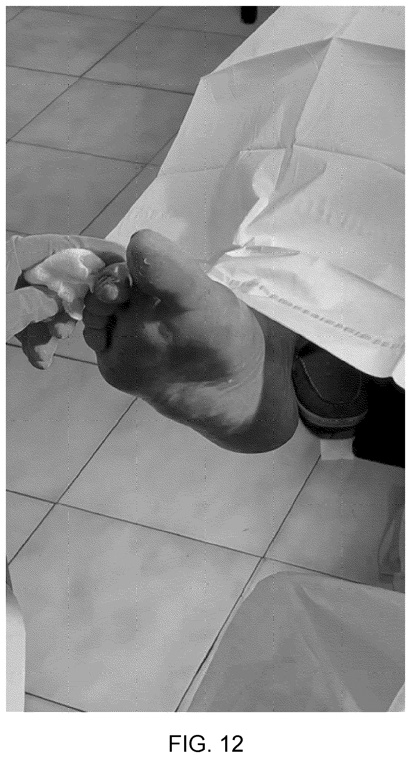

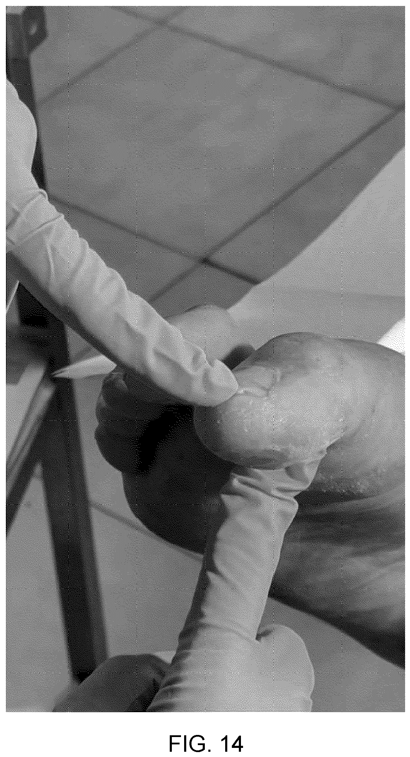

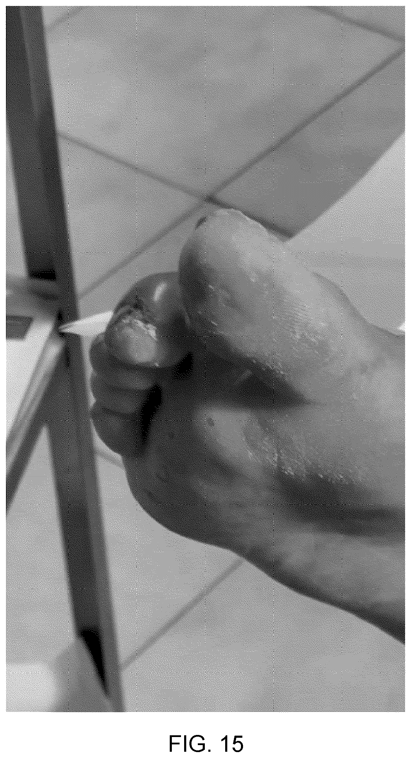

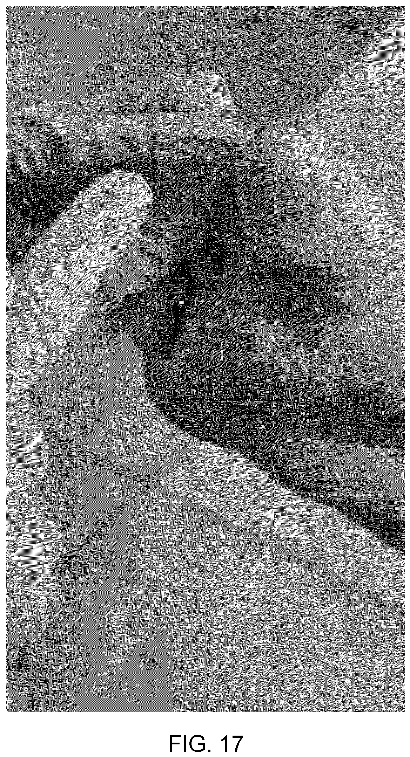

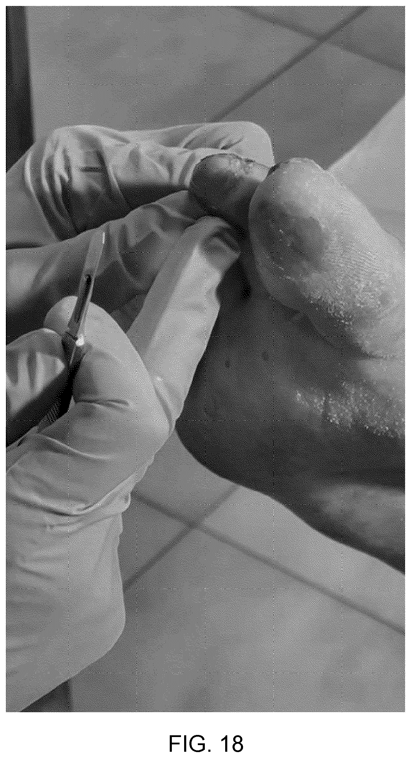

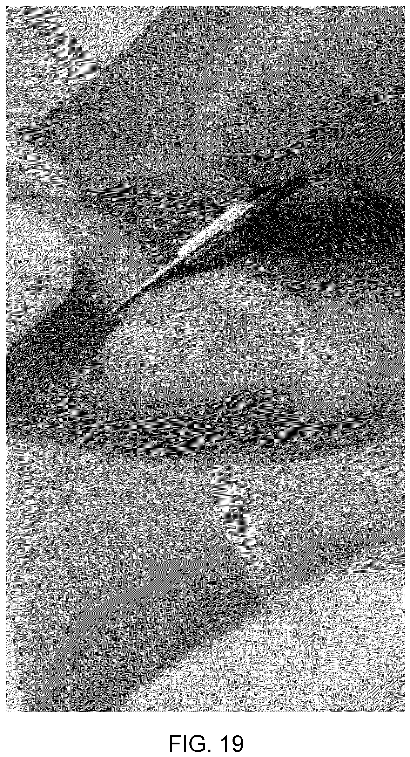

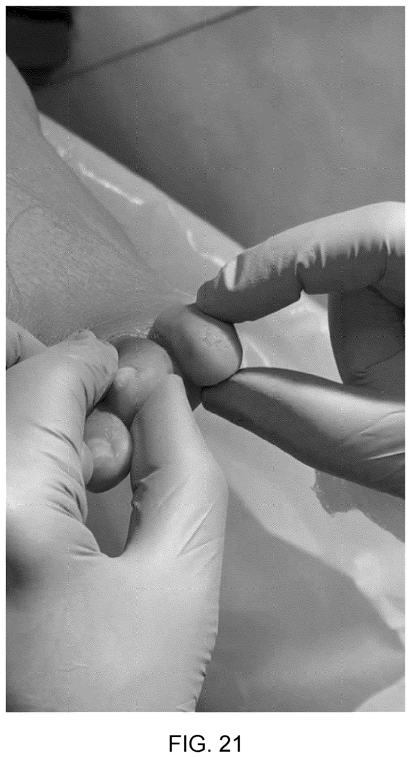

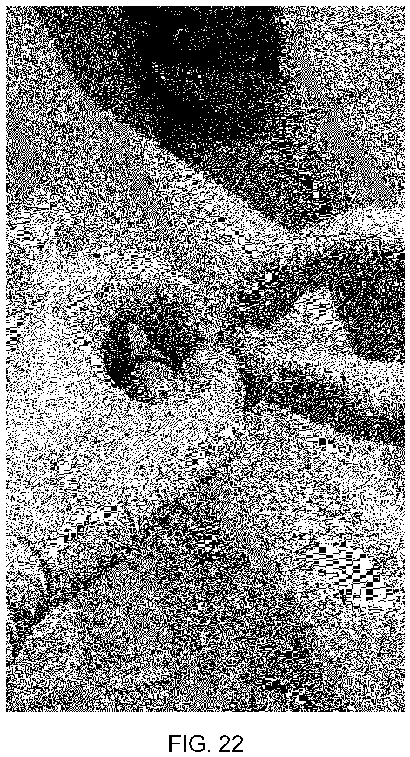

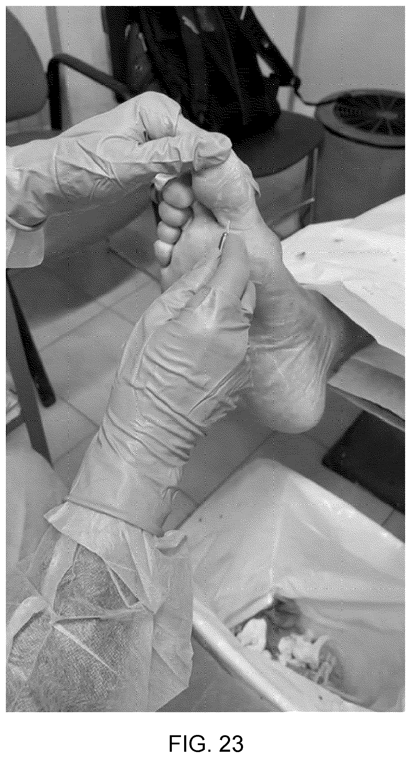

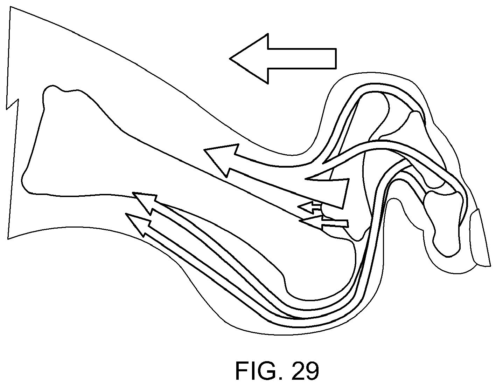

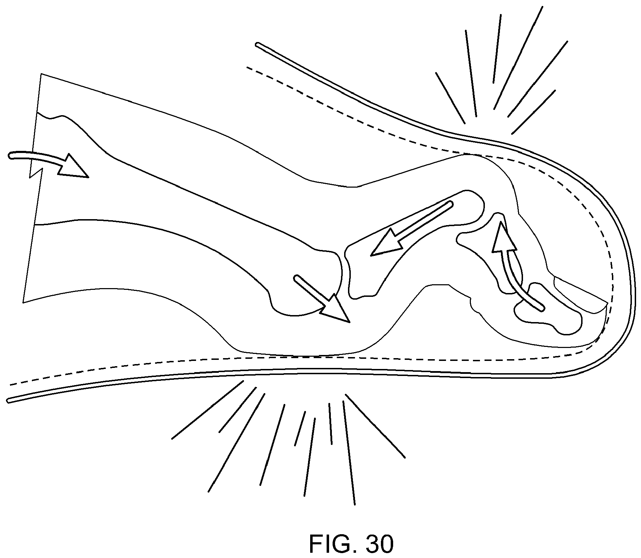

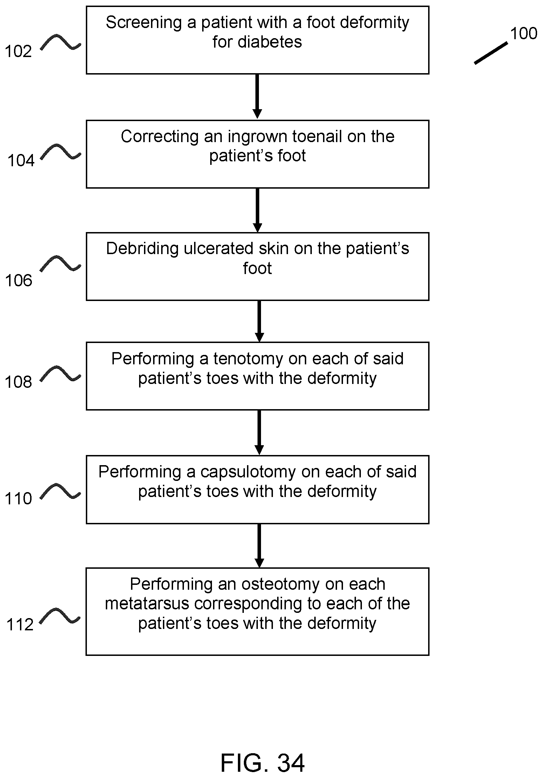

In accordance with embodiments of the invention, a method 100 for correcting common foot disorders associated with diabetes is provided, as illustrated in FIGS. 1-34. As disclosed by the exemplary embodiments described herein, the method 100 treats common foot disorders associated with diabetes by performing sequential treatments on a patient until the condition is treated successfully. Typically, treatment of common foot disorders associated with diabetes focuses on treating symptoms. Embodiments of the method 100 focuses on the cause of the symptoms by releasing pressure on parts of a patient's foot that lead to the resulting symptoms as discussed further below. In embodiments disclosed below, the method treats a patient's foot by first treating one or more tendons, then treats one or more capsules, if necessary, then treats one or more bones, if necessary. One skilled in the art will understand that specific conditions of certain patients may necessitate a variation in sequence and timing of these steps.

The method 100 includes a step 102 of screening a patient with a foot deformity for diabetes. The screening may include but not be limited to glucose testing, hemoglobin A1C testing, confirming prior medical diagnosis, or combinations thereof. If the patient is not diabetic, the method 100 should not continue, as the treatment assumes diabetic neuropathy and poor blood flow. Step 102 may further include diagnostic testing. Diagnostic testing may include but not be limited neuropathic testing, angiopathic testing, visual or x-ray analysis of deformities, or combinations thereof.

If the patient screens positive for diabetes in step 102, the method 100 proceeds to step 104 of correcting any ingrown toenails on the patient's foot. This might include, for example, trimming or removal of the nail. Ingrown toenails are generally infected, and a practitioner should consider treatment prior to moving through further steps of the method 100. It is contemplated that a patient who has been treated for an ingrown toenail may need at least one week of recovery time before additional treatment.

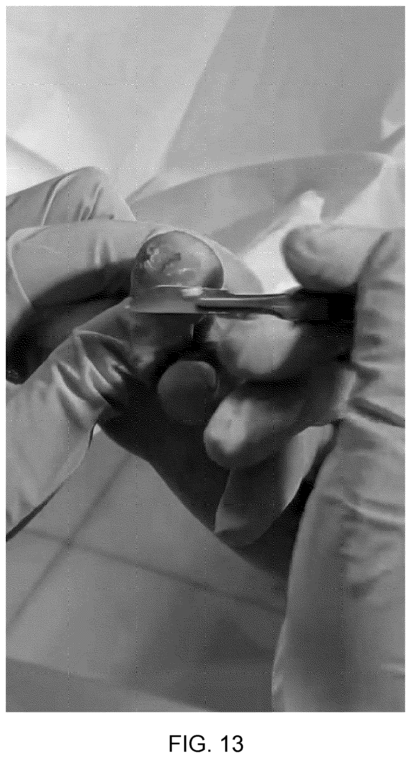

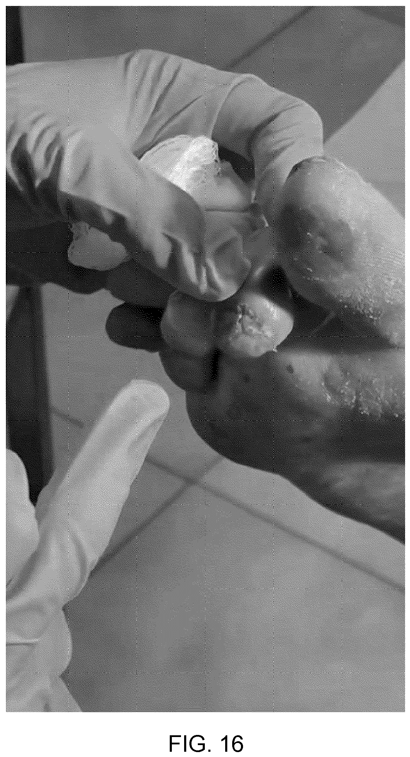

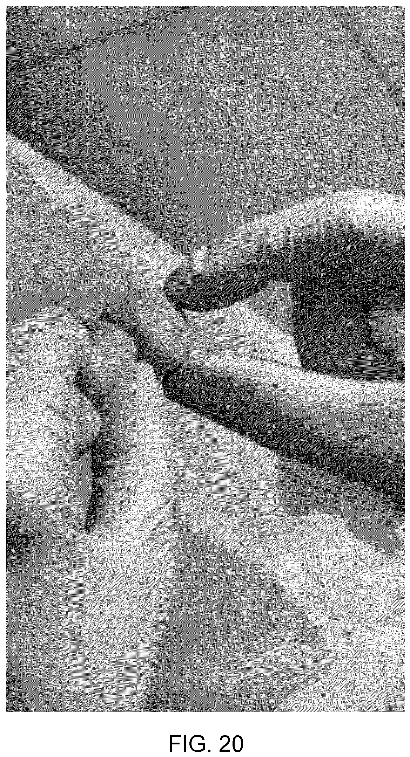

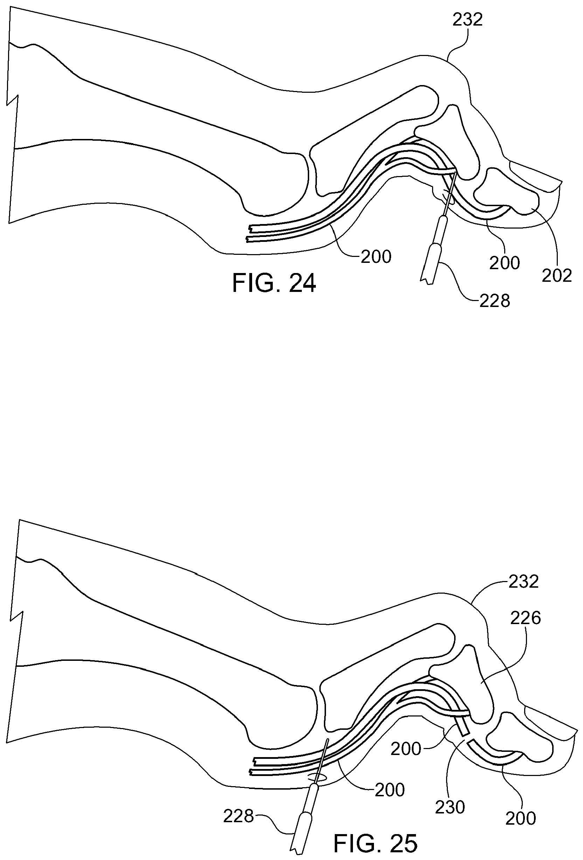

The method 100 includes step 106 of debriding ulcerated skin on the patient's foot, as illustrated in FIGS. 13, 19, and 23. Debriding relates to removal of dead, damaged, or infected tissue to improve the healing potential of remaining healthy tissue. Steps 104 and 106 are corrective steps taken to treat common foot problems experienced by diabetics. These problems stem from loss of feeling due to nerve damage and poor bloodflow. Hammertoes and other misalignments, such as a misaligned or warped metatarsus or hallux rigidus/valgus, tend to develop in diabetic patients and can lead to parts of the toes and feet supporting more weight than normal because of a deformity, as illustrated in FIGS. 24-30. Conditions such as ingrown toenails, ulcers, and interdigital ulcers, for example, occur because of the improper weight distribution on the feet and toes. FIG. 16 illustrates a patient's hammertoe and the resulting ulcer from improper weight distribution. FIG. 20 illustrates an interdigital ulcer between a patient's toes. These conditions affecting the skin and toenails may be treated prior to performing the surgical steps of the method 100. Embodiments of the method 100 are operable for use on patients no matter the condition of their feet, as there is a presumption that the patient has experienced nerve damage, loss of circulation, and likely has an infection.

In step 106, the debridement may be performed by methods known in the art. Removal may be surgical, mechanical, chemical, autolytic (self- digestion), by maggot therapy, or combinations thereof. In podiatry, practitioners such as chiropodists, podiatrists, and foot health practitioners remove conditions such as calluses and verrucae. Surgical or sharp debridement and laser debridement under anesthesia are the fastest methods of debridement. They are very selective, meaning that the person performing the debridement has complete control over which tissue is removed and which is left behind. Surgical debridement can be performed in the operating room or bedside, depending on the extent of the necrotic material and a patient's ability to tolerate the procedure. The surgeon will typically debride tissue back to viability, as determined by tissue appearance and the presence of blood flow in healthy tissue.

The terms tenotomy and capsulotomy refer to the release of tendons and joint capsules. More specifically in relation to hammertoe deformities, the tight tendons and joint capsules located on the top and bottom of the buckled or contracted toe joints are released. And once these tight, soft tissue structures are cut and relaxed, the toe can resume its normal form and function. Embodiments of the method 100 are operable to essentially reverse engineer a deformity, as tightness or restrictions of tissues or bones are released to enable a patient's toes and foot to return to normal form and thereby function normally.

The method 100 therefore includes a step 108 performing a tenotomy on each of said patient's toes with a deformity, as illustrated in FIGS. 1, 3, 4, 6, 8, 9, 23, 24, and 25. A tenotomy is a surgical act which involves the division of a tendon. It may also be referred to as tendon release, tendon lengthening, and heel-cord release. The deformity may be a hammertoe, for example. The tenotomy performed in step 108 may be performed on the ball of the foot and/or under the toes to divide the tendon flexor retinaculum in one or more places depending on the patient's condition, e.g., tightness/stiffness of the toes. Surgical tool 228, such as a scalpel and/or a periosteal elevator may be used. The procedure may also be performed on the top of the foot at the extensor digitorum longus depending on the condition of the patient's foot. As the flexors are stronger than the extensors, patients more frequently experience tightness with the flexors. The incision for the tenotomy may be 5 mm or less.

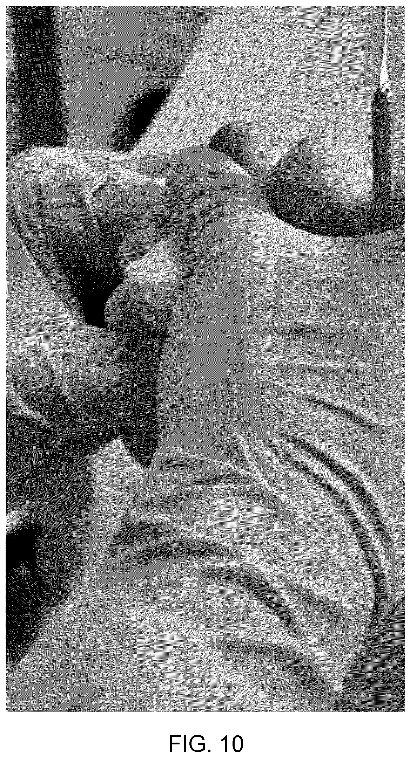

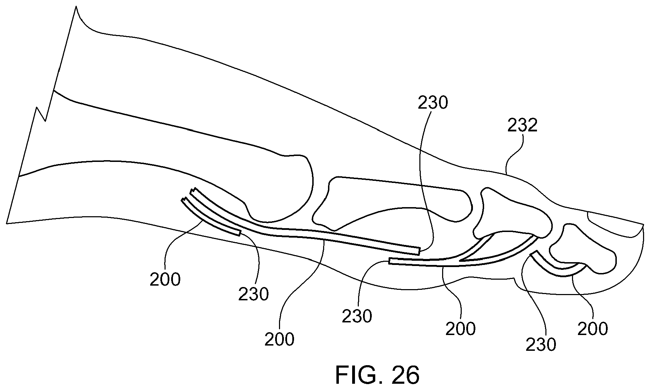

FIG. 24 illustrates a tenotomy of the flexor digitorum longus 200 to release the distal phalanx 202 of the lesser toe 232 experiencing a hammertoe condition. FIG. 25 illustrates a tenotomy of the flexor digitorum longus tendon 200 to release the medial phalanx 226 of the lesser toe 232. FIG. 26 illustrates the corrected lesser toe 232 with released tendon ends 230 no longer in a hammertoe position after the tenotomies illustrated in FIGS. 24-25. The divided tendon will regenerate after the procedure. In one embodiment of the method, a surgeon will perform one tenotomy and manually stretch the toe (FIG. 10) and visually inspect the toe to confirm whether the toe has been released from a tight condition such as a hammertoe. If the toe continues to be restricted, the surgeon may perform subsequent tenotomies and/or capsulotomies (as described below) and/or manual stretching until the condition is released.

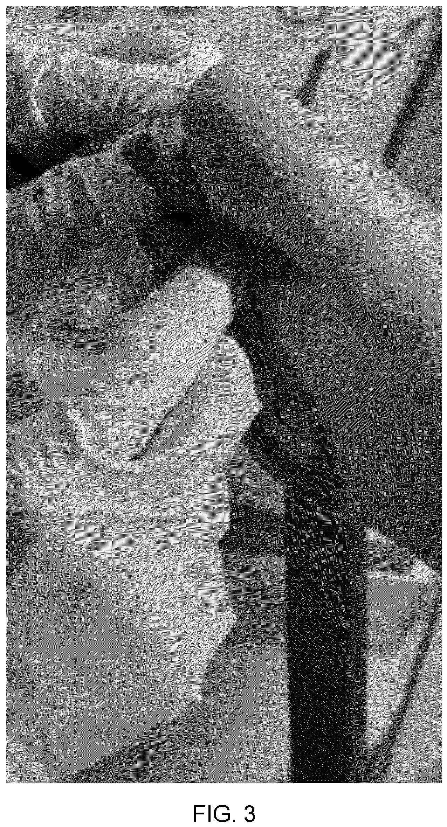

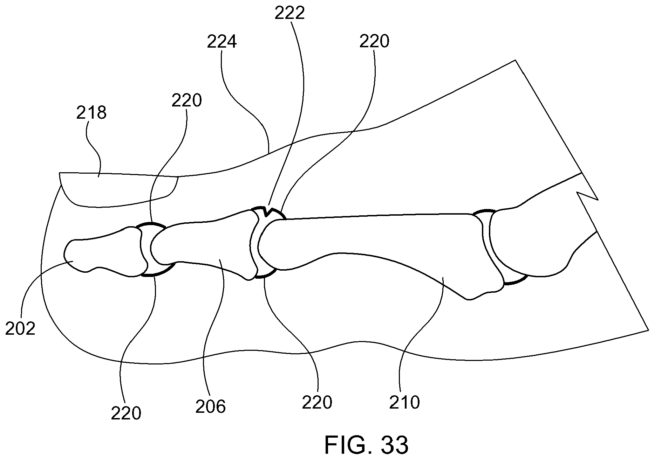

The method 100 may therefore include a step 110 of performing a capsulotomy, as illustrated in FIGS. 3, 4, 8, 9, 23, and 33, on each of the patient's toes with a deformity if the tenotomy performed in step 108 did not correct the deformity. The capsulotomy may be performed in the same incision as the tenotomy in step 108. As illustrated in FIG. 33, the capsulotomy 222 punctures the joint capsule 220 of great toe 218 via incision 224 and dissects the joint capsule 220 to widen the fibrous membrane of the joint capsule 220 by about 5 mm dorsally to allow the toe to straighten. By opening the capsule, which is generally inflexible, the surgeon can manipulate the tissues and stretch the toe (FIG. 10) such that the affected toe will become level with the other toes. The capsulotomy in step 110 is performed when the tenotomy did not correct the deformity. The deformity is not corrected if the digit is not in line, or all the toes are not in the same plane. Confirmation may be made through observation and/or x-ray.

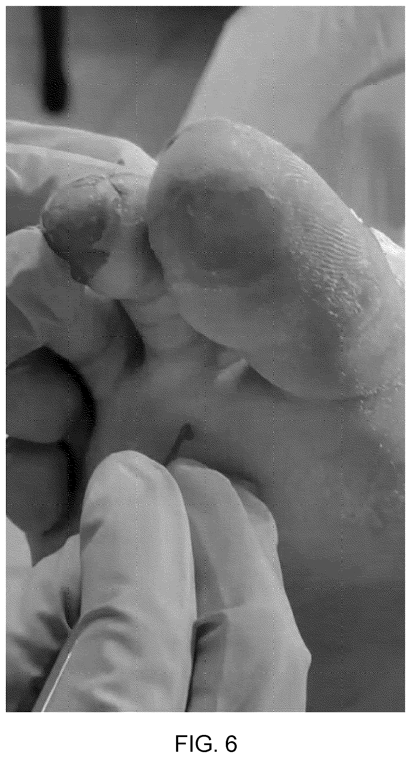

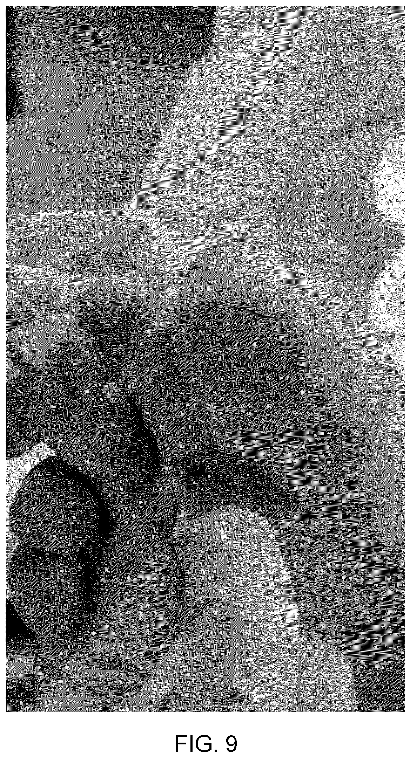

A surgeon performing the method 100 will evaluate the condition of the patient and the efficacy of each tenotomy in step 108 and/or capsulotomy in step 110 to determine the success of the procedure through these steps, wherein success is the release of a restricted toe that returns to normal form and function. For example, a surgeon may perform a tenotomy on the ball of a patient's foot, as illustrated in FIG. 6, then stretch the toe as illustrated in FIG. 10, but determine that the condition, a hammertoe as illustrated, has not been corrected. Next, the surgeon may perform a second tenotomy under the toe, as illustrated in FIG. 9, then stretch the toe, then observe visually if the condition has been corrected. At this step in this example, the surgeon, based on the condition of the patient, may reenter the same incision as the second tenotomy in FIG. 9 and perform a capsulotomy on the affected toe, then stretch the toe. The surgeon will continue this process of tenotomies and/or capsulotomies along the toe, either on the plantar or dorsal sides of the toe, until the condition is released. If the condition is not released, an osteotomy may be required. However, if the bones are not deformed, one or more tenotomies or capsulotomies may need to be repeated because it is likely that the procedure did not properly release the tendon or the capsule, as the completion of these steps will release the toe if performed correctly.

The method 100 preferably will begin with the most affected joint first and then move to the lesser affected joints. In one embodiment, the method starts distally (towards the end of the toe) and moves proximally (towards the ankle) until the toe is released. The order of these steps might be reversed if there is a damaged/symptomatic issue in a location that would normally receive a treatment. Thus, a capsulotomy might be performed prior to a tenotomy if the patient's condition necessitates it. For example, a patient may have an infected ulcer on the ball of their foot which would prevent a tenotomy in that location. Rather than waiting, the procedure should begin in an alternative location and capsulotomies may be performed prior to tenotomies. This is because the pressure on the foot needs to be released. Decompression of the foot is critical and time sensitive because the pressure causes cell death. By releasing the pressure, the process of regeneration can begin in the affected area. After a tenotomy in step 108 and/or a capsulotomy in step 110, a surgeon will manually stretch the affected toes to assist with loosening the toes to return the toes to their normal positions, as illustrated in FIG. 10.

The method 100 includes a step 112 of performing an osteotomy on each metatarsus corresponding to each of the patient's toes with a deformity if the tenotomy performed in step 108 and the capsulotomy performed in step 110 did not correct the deformity.

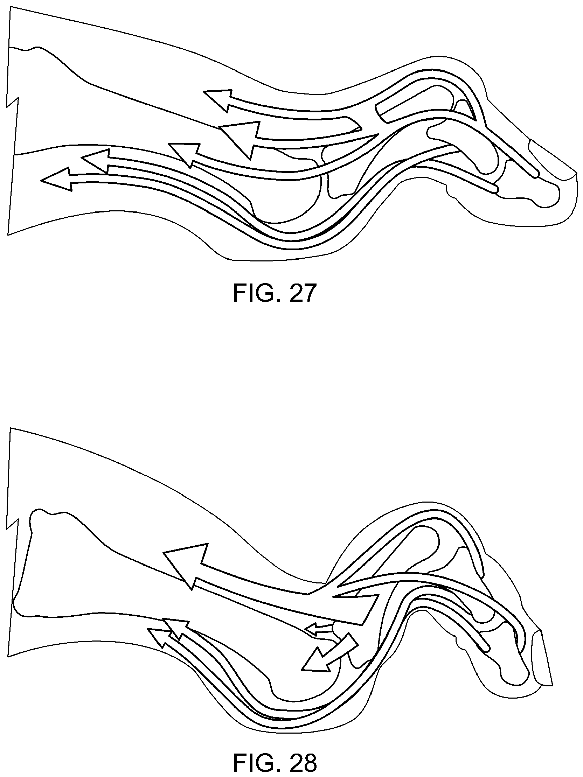

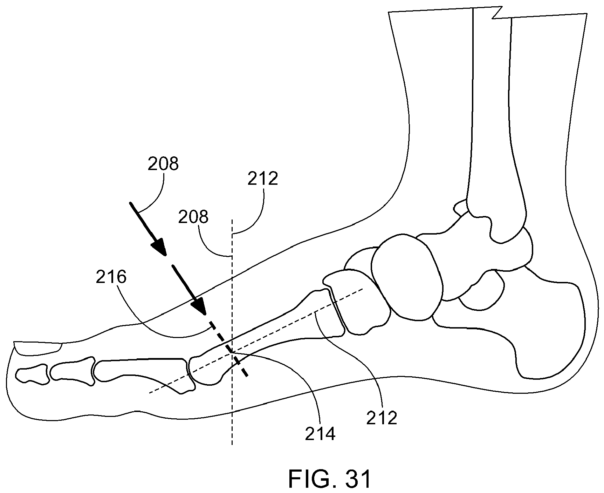

In one embodiment of the method 100, the osteotomy in step 112 includes the step of cutting a keyhole incision on the top of the patient's foot dorsal and proximal to a surgical neck of a metatarsal head of the metatarsus. As illustrated in FIGS. 31-32, the osteotomy 216 in step 112 includes the step of drilling a hole through the center of metatarsus 210 at an angle 208 such that the drill goes through the metatarsus 210 cortex to cortex. The osteotomy 216 is then performed with a side cutting drill bit laterally then medially from the midline of the keyhole until the osteotomy 216 is complete. By drilling into the metatarsus 210 first, the drill bit creates an access point for the side cutting drill bit to cut from the middle of the metatarsus 210 to the edge of the metatarsus 210. This procedure avoids the normal dissection of the tissues surrounding this area, such as tendons, ligaments, blood vessels, nerves, muscle, fascia, and subcutaneous tissue. The angle 208 ranges from 35 degrees to 45 degrees periosteum (relative to vertical line 212), with 40-45 degrees most preferable, enabling the surgeon to drill into the metatarsus 210 perpendicularly to centerline 214. The osteotomy in step 112 is performed in a dorsal distal to plantar proximal plane to the metatarsal head. The osteotomy in step 112 may further include the step of manually confirming by visual inspection or by x-ray that each head of the metatarsus 210 is lined up together. Upon proper anatomical correction, a surgeon will note that the metatarsal heads are now level relative to each other. The plantar flexed metatarsal is as a result of the deforming forces on the forefoot and neuropathic changes that allow the hammer toe syndrome and the contracted fascia and ankle equinus, as illustrated in FIGS. 27-30. These forces can lead to the deformation of the metatarsus 210.

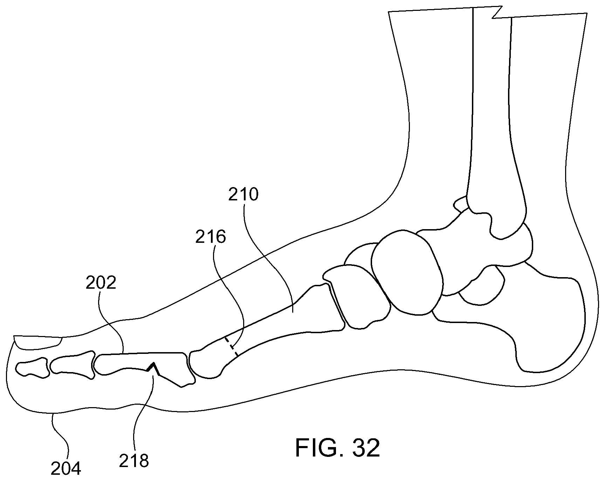

An object of the osteotomy is to sever the affected metatarsus. The pressure of the rest of the structure of the foot keeps the two parts of the severed metatarsus in place while the bone reforms without screws or pins. Because of the operation at an angle, the bone is cut straight through, and one section of the bone will slide up relative to the other section of the bone. The outside cortex of the sections of the bones will contact and hold to each other. A new cellular matrix is formed as a result of the acute injury caused by severing the bone, thereby stimulating stem cells and chemotaxis, healing the bone in the corrected position. The adjustment to the position of the bone is minor, typically ranging from 2 mm to 5 mm. In one embodiment of the method 100, the osteotomy in step 112 is a wedge osteotomy to the proximal phalanx 206 of the great toe 204, as illustrated in FIG. 32, where a portion of the bone, most typically a wedge-shaped portion, is removed to further increase the amount of corrective adjustment. FIG. 32 illustrates a wedge osteotomy 218 of a proximal phalanx 202 of a great toe 204. An osteotomy or a wedge osteotomy in step 112 may be performed on various bones of the foot depending on the patient's condition and the degree of correction required for the patient.

A purpose of the osteotomy in step 112 is the correction of the deformity. This correction can be confirmed anatomically or functionally. The incision may be made dorsal and proximal to the surgical neck of the metatarsal head, for example, as illustrated in FIG. 31. A 64 MIS beaver blade may be used for sharp dissection to the metatarsal head. The instruments may be held at an angle ranging from 35 degrees to 45 periosteum elevated at the same site. A keyhole incision may be made to the surgical neck at the midline of the metatarsal. The osteotomy may be performed through and through with use of an Osada bone cutting drill, for example. Utilizing a drill has advantages over a saw. The surgery can be performed with a small incision, such as a 0.5 cm window. The spinning of the drill bit on one axis, as opposed to the motion of a saw, gives the surgeon control of the drill and the ability to avoid hitting tendons or vessels surrounding the affected metatarsus. This procedure reduces human error of open surgery. Further, closing an incision on the top of a foot large enough to accommodate a saw is challenging. Traditionally, doctors have been instructed not to operate on a diabetic foot unless the patient is septic or critical, as larger incisions do not close because of the diabetic complications. Here, the incisions of the tenotomy, capsulotomy, and osteotomy are small enough to avoid issues with closure and healing associated with diabetic complications. The success of the osteotomy may be confirmed by x-ray or manually by seeing that the metatarsal heads are lined up together. The entire procedure may be done in a plane to the metatarsal head that is described as dorsal distal to plantar proximal. After the osteotomy, the stimulation of stem cells from the bone marrow and chemotaxis to the area enhance the healing process. Closure of the incision may be done with a 3.0 vicryl suture and the foot is dressed to offload the area and protect the tissues. A cast or surgical shoe may be used post-op.

To determine the success of the procedure, a practitioner may objectively evaluate the success via visual observation of the foot and toes to see if they are straight and in the same plane or misaligned or abnormally shaped. The practitioner may also look for evidence of recurring or new ulcers. An x-ray may also be utilized to confirm the patient's bones are straight and the toes line up. Not all steps are necessary depending on the location and severity of the issues. For example, some patients may only require debriding ulcerated skin and a tenotomy to correct the deformities and capsulotomies or osteotomies are not necessary.

It should be understood that modifications to the embodiments disclosed herein can be made to meet a particular set of design criteria. For instance, the number of or configuration of components or parameters may be used to meet a particular objective.

It will be apparent to those skilled in the art that numerous modifications and variations of the described examples and embodiments are possible in light of the above teachings of the disclosure. The disclosed examples and embodiments are presented for purposes of illustration only. Other alternative embodiments may include some or all of the features of the various embodiments disclosed herein. For instance, it is contemplated that a particular feature described, either individually or as part of an embodiment, can be combined with other individually described features, or parts of other embodiments. The elements and acts of the various embodiments described herein can therefore be combined to provide further embodiments.

It is the intent to cover all such modifications and alternative embodiments as may come within the true scope of this invention, which is to be given the full breadth thereof. Additionally, the disclosure of a range of values is a disclosure of every numerical value within that range, including the end points. Thus, while certain exemplary embodiments of the device and methods of making and using the same have been discussed and illustrated herein, it is to be distinctly understood that the invention is not limited thereto but may be otherwise variously embodied and practiced within the scope of the following claims.

## Claims

1. A method for correcting a foot disorder, comprising:
screening a patient with a foot deformity for diabetes;
optionally correcting an ingrown toenail on said patient's foot;
optionally debriding ulcerated skin on said patient's foot;
performing a tenotomy on each of said patient's toes with the deformity;
performing a capsulotomy on each of said patient's toes with the deformity if said tenotomy did not correct said deformity; and
performing an osteotomy on each metatarsus corresponding to each of said patient's toes with the deformity if said tenotomy and said capsulotomy did not correct said deformity.

2. The method of claim 1, wherein the tenotomy comprises dividing a tendon flexor retinaculum.

3. The method of claim 1, wherein the tenotomy comprises dividing an extensor digitorum longus.

4. The method of claim 1, further comprising manually stretching said patient's foot after the step of performing a tenotomy.

5. The method of claim 1, further comprising performing a second tenotomy on each of said patient's toes with the deformity if said tenotomy did not correct said deformity.

6. The method of claim 1, wherein the capsulotomy comprises puncturing and dissecting a joint capsule.

7. The method of claim 1, further comprising manually stretching said patient's foot after the step of performing a capsulotomy.

8. The method of claim 1, wherein said osteotomy comprises:
cutting a keyhole incision on the top of said patient's foot dorsal and proximal to a surgical neck of a metatarsal head of said metatarsus; and
dissecting said metatarsus,
wherein said dissection is performed at an angle such that said metatarsus is dissected perpendicularly to a centerline of said metatarsus.

9. The method of claim 8, wherein said angle ranges from 35 degrees to 45 degrees periosteum.

10. The method of claim 8, wherein said osteotomy is performed in a dorsal distal to plantar proximal plane to said metatarsal head.

11. The method of claim 1, wherein said osteotomy is a wedge osteotomy.

12. The method of claim 8, wherein said osteotomy further comprises the step of manually confirming by visual inspection or x-ray that each head of said metatarsus is lined up together.

13. The method of claim 1, wherein said deformity is hammertoe.

14. The method of claim 1, wherein the step of screening a patient with a foot deformity for diabetes comprises a screening method selected from the group consisting of glucose testing, hemoglobin A1C testing, testing to confirm a prior diagnosis, and combinations thereof.

15. The method of claim 1, wherein the step of screening the patient with the foot deformity for diabetes comprises a diagnostic testing method selected from the group consisting of neuropathic testing, angiopathic testing, visual analysis of the deformity, x-ray analysis of the deformity, and combinations thereof.
